# EUROPEAN PATENT SPECIFICATION

(11) **EP 4 253 512 B1**
(45) Date of publication and mention of the grant of the patent: **28.10.2026**
(21) Application number: 22382292.5
(22) Date of filing: 29.03.2022
(51) Int. Cl.: C11D 3/48, A01N 25/00, A01P 1/00, A61L 2/16, C11D 3/382, C11D 3/14, C11D 11/00

(54) **SANITIZING PRODUCT FOR CLEANING AND DISINFECTING ANIMAL FEEDING TUBES**
DESINFEKTIONSMITTEL ZUM REINIGEN UND DESINFIZIEREN VON FUTTERROHREN
PRODUIT DE DÉSINFECTION POUR NETTOYER ET DÉSINFECTER DES TUBES D'ALIMENTATION POUR ANIMAUX

(43) Date of publication of application: 04.10.2023
(73) Proprietor: Garcia Paez, David, 14546 Santaella (Córdoba) (ES)
(72) Inventor: Garcia Paez, David, 14546 Santaella (Córdoba) (ES)
(74) Representative: Pons IP

(56) References cited:
- EP-A1- 2 631 286
- WO-A1-2019/195403
- US-A1- 2019 350 198

## Description

The present invention relates to a completely natural product for cleaning and disinfecting animal feeding systems, which are very difficult to access for cleaning and sanitising because of their assembly, based on a combination of olive pit and purple garlic. Therefore, the invention could fall within the field of cleaning products, and more particularly, products intended for disinfecting pipes.

### STATE OF THE ART

In recent years, the livestock sector is becoming more aware of the fact that it is more cost-effective to prevent pathologies than to treat them; to do so, it is necessary to reduce the presence of microorganisms that can interact with livestock, weakening them and reducing their productivity and even causing their death.

The condition and sanitation of feeding circuits is therefore essential to prevent the emergence of a microbial population that can grow on an inert or living surface.

These cleaning and disinfection operations are therefore actions whose purpose is to minimise or eliminate the presence of dirt and pathogenic microorganisms, leaving a minimal presence of detergent and disinfectant residues. The cleaning and disinfection actions must be considered basic and essential actions in the factory and the result of these operations affects the final quality of the product.

Usually, the animal feed at these agricultural and livestock farms comes from silos located outside the barn where the animals live; from these silos, the feed is passed to the inside where it is distributed towards the feed lines and reaches the feeding troughs by the action of a flexible auger that is moved by an electric motor at one end of the line.

Due to humidity and temperature on the farm, the feed cakes and gets embedded into the inner walls of the tube, obstructing the outlet to the feeding troughs and making it difficult for the feed to pass along the line, such that less feed reaches the animals. The cleaning thereof was performed by disassembling the flexible auger and pulling it out with a disinfectant product and a brush, to which extensions are attached, and cleaning is carried out by dragging, which does not ensure the elimination of fungi, bacteria and insects.

The cost of conventional cleaning is high due to the mere fact that equipment must be disassembled and, in the case the auger is not removed, the cleaning would be neither complete nor effective.

Moreover, the materials used for cleaning and disinfecting must be adapted to the objectives to be achieved and they must have the condition of food use in order to avoid any problem related to human or animal health.

Today, the most popular cleaning compositions, such as particulate compositions or liquid compositions (including those of the gel or paste type) that contain abrasive components, are based on abrasive particles with shapes that vary from spherical to irregular. The most common abrasive particles are inorganic, such as carbonate salt, clay, silica, silicate, slate ash, perlite and quartz sand, or organic polymer beads, like polypropylene, PVC, melamine, urea, polyacrylate and derivatives thereof, and they take the form of a creamy liquid composition with the abrasive particles suspended therein.

Furthermore, abrasive particles derived from natural materials such as nutshells, for example, walnut shells and almond shells, or those derived from the pits of the seeds, for example, apricot pits and cherry pits, sometimes meet the aforementioned requirements, but they appear in nature with a dark colour and their inclusion in a cleaning product results in an unsightly liquid composition with a muddy appearance. This is highly undesirable to consumers/users because it compromises the appearance of the liquid composition and its cleaning performance. Therefore, there is a real need to identify an abrasive particle derived from a natural material that meets both aesthetic and performance requirements for the liquid cleaning composition.

Likewise, olive pits and seeds are an important by-product generated in the olive oil extraction and pitted table olive industries. As lignocellulosic material, hemicellulose, cellulose and lignin are the main components of the olive pit, in addition to the composition of proteins, fats, phenols, free sugars and polyols. The main use of this biomass is combustion to produce electrical energy or heat. Other uses have been mentioned such as activated carbon, furfural production, plastic filling, abrasives and cosmetics or other potential uses as a biosorbent, animal feed or resin formation (see, for example, EP2631286A1, WO2019195403A1 and US2019350198A1).

Likewise, the benefits of purple garlic, and in particular the antimicrobial properties thereof, are known, so the use thereof in different livestock systems improves the immune system, since, unlike conventional antibiotics, purple garlic is a selective bactericide that improves and stimulates the intestinal flora of animals against pathogenic bacteria.

Considering this state of the art, an aim of the present invention is to provide a liquid cleaning composition suitable for cleaning pipes and other hard-to-reach surfaces that are chemical-free, biodegradable and specially adapted for use in the environment of agricultural and livestock farms.

### DESCRIPTION OF THE INVENTION

In the present invention, a completely natural product has been developed, which is devoid of any chemical product, sanitises, cleans and disinfects feeding systems with tubes for feeding farm animals, which are very difficult to access for cleaning and sanitising because of their assembly, based on a combination of olive pit and purple garlic.

Some advantages of the compositions of the invention include:
- They have good drag capacity thanks to the particle size between 5 and 7 mm;
- They are non-caking and do not expire under optimal storage conditions;
- Due to their additives, they are disinfectants and anti-insect agents;
- Free of impurities and any toxic element or hazardous residue that releases harmful gases;
- Not palatable for animal intake, although if ingested it is completely safe;
- Homogeneous at all times of the year;
- Easy handling for the applicator;
- Biodegradable and suitable for transformation into organic fertiliser and/or energy production.

Indeed, the easy and practical use thereof is worth noting since it does not require disassembling the feeding system in agricultural and livestock farms and it has a one hundred percent natural origin.

Therefore, a first aspect of the invention relates to a sanitising composition according to claim 1, in particular characterised in that it comprises dry olive pit in a percentage by weight greater than 90% of the total and fresh purple garlic in a percentage by weight between 0.5 and 1.0% of the total, and wherein the other components are silica, sodium chloride and acidulants until 100% of the total weight is reached.

In a preferred embodiment, the percentage by weight of fresh purple garlic is between 0.5 and 0.7% w/w, and more preferably it is 0.65% w/w.

In another preferred embodiment, the percentage by weight of silica is between 0.2 and 0.3% w/w, and more preferably it is 0.28 % w/w.

In another preferred embodiment, the percentage by weight of sodium chloride is in a percentage between 0.02 and 0.07% w/w, and more preferably it is 0.05 % w/w.

In another preferred embodiment, the acidulant is selected from citric acid and citric acid extract, or the combination thereof.

In another preferred embodiment, the percentage by weight of citric acid is between 0.005 and 0.015% w/w, and more preferably it is 0.01% w/w.

In another preferred embodiment, the percentage by weight of citric extract is between 0.005 and 0.015% w/w, and more preferably it is 0.01% w/w.

Also disclosed herein is a method for obtaining the core of dry olive pit, characterised in that it comprises the following steps:
i) Dehydration of the olive cake until the relative humidity thereof drops below 10.5%;
ii) Vibratory screening of the dry olive cake from step i) to separate particles with a size comprised between 3 and 7 mm;
iii) Removal of dust from the particles from step ii) by passing said particles through a duct that narrows causing dust to be collected by the Venturi effect.

In another aspect, the invention relates to the use of the sanitising composition as described above to clean the feeding ducts of farms. Also disclosed herein is the use of the sanitising composition for removing the material embedded inside the feed line, reducing the bacterial and fungal load, and acting as an anti-insect agent.

The term "citric acid extract" in the present invention refers to a combination of extracts from fruit of the citrus genus selected from citrus paradisi (grapefruit), citrus aurantium bergamia (bergamot), citrus sinensis (orange), and citrus reticulata (tangerine), in vegetable glycerine.

The term "antimicrobial" comprises a series of particular agents acting against specific forms of microorganisms, such as bactericides (antibacterial agents), fungicides (antifungal agents), insect-proof agents, moth-proof agents, herbicides, algaecides, agents against decomposition, and products that fight dust microorganisms.

Throughout the description and the claims, the word "comprises" and its variants are not intended to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

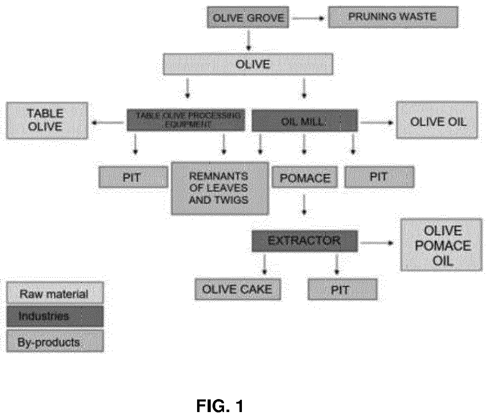

**Fig. 1** shows the steps in the process for obtaining the olive pit.

### EXAMPLES

Next, the invention will be illustrated by means of assays carried out by the inventors which highlights the usefulness of the sanitising composition of the invention.

### Example 1. Method for obtaining the olive pit

To produce olive oil, after the olives are harvested, the olive is crushed whole, in other words, with the pit. The olive oil is pressed and extracted using physical processes. Olive cake, i.e., the solid residue of the olive (skin, pulp and pit fragments), are processed again, in a wet state, by means of specialised machines (centrifuges) to newly extract oil, thus obtaining the by-products themselves.

To dehydrate the olive cake, it is circulated through a trommel for 50 minutes at 140 degrees Celsius to lower the relative humidity thereof to 10.26%. It is sorted and selected using vibratory screening, depending on the granulometries thereof, from 3 to 7 mm. The dust is extracted using a Venturi-type collection system and stored in big bags until use. The result is a light-beige to medium-tan calibrated granule, with a neutral odour and no dust or residue.

With the pit clean and calibrated between 5 and 7 mm, the exact amounts of pit are combined with the formulated product in an industrial mixer for 15 min for every 900 kilos of mixture at room temperature and pressure.

Once this process is finished, the mixture passes by mechanical means to an automated packaging machine in which 15 kg Kraft paper bags will be filled with the finished product. They will be placed on pallets avoiding contact with the ground and the process is finished with the netting, lamination and storage of the packages, leaving them ready for transport.

| **Name of Measurement** | **Minimum value** | **Maximum value** | **Mean value** | **Units** |
|---|---|---|---|---|
| BASIC ANALYSIS | | | | |
| Humidity (w.b.) | 9.01 | 22.67 | 15.47 | % |
| Ashes (w.b.) | 0.50 | 2.00 | 1.37 | % |
| Volatiles (d.b.) | 76.40 | 83.31 | 79.86 | % |

| ELEMENTAL ANALYSIS | | | | |
|---|---|---|---|---|
| Carbon (d.b.) | 54.71 | 55.60 | 55.16 | % |
| Hydrogen (d.b.) | 5.80 | 5.95 | 5.88 | % |
| Sulphur (d.b.) | 0.01 | 0.05 | 0.03 | % |
| Chlorine (d.b.) | 0.02 | 0.06 | 0.04 | % |
| Oxygen | 41.55 | 42.74 | 42.15 | % |

| CALORIFIC VALUE | | | | |
|---|---|---|---|---|
| Higher Calorific Value (d.b.) | 4,778 | 5,203 | 4,968 | Kcal/kg |
| Lower Calorific Value (d.b.) | 3,806 | 4,801 | 4,318 | Kcal/kg |
| Higher Calorific Value (w.b.) | 4,356 | 4,632 | 4,493 | Kcal/kg |
| Lower Calorific Value (w.b.) | 3,477 | 4,064 | 3,854 | Kcal/kg |

| | | | | |
|---|---|---|---|---|
| Note: d.b. = dry basis, w.b.= wet basis | | | | |

### Example 2. METHOD FOR CLEANING THE feeding ducts

As mentioned earlier, the conventional cleaning of feeding systems on agricultural and livestock farms was carried out by disassembling the flexible auger and pulling it out with a disinfectant product and a brush, to which extensions are attached, and cleaning is carried out by dragging, which does not ensure the elimination of fungi, bacteria and insects.

The composition of the invention provides an all-in-one cleaning alternative, where it does not have to be disassembled (reducing time and costs), and it cleans, disinfects, debugs and descales both the auger and the line that contains it in its path.

For use thereof, the line is lowered to the ground, the feeding troughs are rotated 90 degrees so that the product does not fall therein and the outlet is left free at the other end of the line; with the machine running, in the hopper that supplies feed to the line, 2 bags (15 Kg per bag) of the composition of the invention are poured constantly and progressively so that blockage do not occur. The product is left to work until all of it has come out the other end, descaling adhered material and thus cleaning the duct.

Once the treatment is finished, the line of feeding troughs is raised and the by-product generated, olive pit together with remnants of feed, is collected, material that by being biodegradable can be composted with the rest of the organic matter or used to generate energy by burning it in a boiler.

## Claims

1. A sanitising composition **characterised in that** it comprises olive pit in a percentage by weight greater than 90% of the total weight and purple garlic in a percentage by weight between 0.5 and 1.0% of the total weight, and wherein the other components are silica, sodium chloride and acidulants until 100% of the total weight is reached, wherein the olive pit has a particle size comprised between 5 and 7 mm.

2. The composition according to claim 1, wherein the percentage by weight of purple garlic is between 0.5 and 0.7% w/w.

3. The composition according to the preceding claim, wherein the percentage by weight of purple garlic is 0.65% w/w.

4. The composition according to any of the preceding claims, wherein the percentage by weight of silica is between 0.2 and 0.3% w/w.

5. The composition according to the preceding claim, wherein the percentage by weight of silica is 0.28% w/w.

6. The composition according to any of the preceding claims, wherein the percentage by weight of sodium chloride is between 0.02 and 0.07% w/w.

7. The composition according to the preceding claim, wherein the percentage by weight of sodium chloride is 0.05% w/w.

8. The composition according to any of the preceding claims, wherein the acidulant is selected from citric acid and citric acid extract.

9. The composition according to the preceding claim, wherein the acidulant is a combination of citric acid and citric acid extract.

10. The composition according to claim 8, wherein the percentage by weight of citric acid is between 0.005 and 0.015% w/w.

11. The composition according to the preceding claim, wherein the percentage by weight of citric acid is 0.01% w/w.

12. The composition according to claim 8, wherein the percentage by weight of citric acid extract is between 0.005 and 0.015% w/w.

13. The composition according to the preceding claim, wherein the percentage by weight of citric acid extract is 0.01% w/w.

14. Use of a composition according to any of claims 1 to 13 to clean the feeding ducts of farms.

## Patentansprüche

1. Desinfizierende Zusammensetzung, **dadurch gekennzeichnet, dass** sie Olivenkerne in einem Gewichtsanteil von mehr als 90 % des Gesamtgewichts und lila Knoblauch in einem Gewichtsanteil zwischen 0,5 und 1,0 % des Gesamtgewichts umfasst, und wobei die übrigen Bestandteile Kieselsäure, Natriumchlorid und Säuerungsmittel sind, bis 100 % des Gesamtgewichts erreicht sind, wobei die Olivenkerne eine Partikelgröße zwischen 5 und 7 mm aufweisen.

2. Zusammensetzung nach Anspruch 1, wobei der Gewichtsanteil an lila Knoblauch zwischen 0,5 und 0,7 % w/w liegt.

3. Zusammensetzung nach dem vorhergehenden Anspruch, wobei der Gewichtsanteil an lila Knoblauch 0,65 % w/w beträgt.

4. Zusammensetzung nach einem der vorhergehenden Ansprüche, wobei der Gewichtsanteil an Kieselsäure zwischen 0,2 und 0,3 % w/w liegt.

5. Zusammensetzung nach dem vorhergehenden Anspruch, wobei der Gewichtsanteil an Kieselsäure 0,28 % w/w beträgt.

6. Zusammensetzung nach einem der vorhergehenden Ansprüche, wobei der Gewichtsanteil an Natriumchlorid zwischen 0,02 und 0,07 % w/w liegt.

7. Zusammensetzung nach dem vorhergehenden Anspruch, wobei der Gewichtsanteil an Natriumchlorid 0,05 w/w beträgt.

8. Zusammensetzung nach einem der vorhergehenden Ansprüche, wobei das Säuerungsmittel aus Zitronensäure und Zitronensäureextrakt ausgewählt ist.

9. Zusammensetzung nach dem vorhergehenden Anspruch, wobei das Säuerungsmittel eine Kombination aus Zitronensäure und Zitronensäureextrakt ist.

10. Zusammensetzung nach Anspruch 8, wobei der Gewichtsanteil an Zitronensäure zwischen 0,005 und 0,015 % w/w liegt.

11. Zusammensetzung nach dem vorhergehenden Anspruch, wobei der Gewichtsanteil an Zitronensäure 0,01 % w/w beträgt.

12. Zusammensetzung nach Anspruch 8, wobei der Gewichtsanteil an Zitronensäureextrakt zwischen 0,005 und 0,015 % w/w liegt.

13. Zusammensetzung nach dem vorhergehenden Anspruch, wobei der Gewichtsanteil an Zitronensäureextrakt 0,01 % w/w beträgt.

14. Verwendung einer Zusammensetzung nach einem der Ansprüche 1 bis 13 zur Reinigung der Futterleitungen in landwirtschaftlichen Betrieben.

## Revendications

1. Composition assainissante **caractérisée en ce qu'**elle comprend du noyau d'olive dans un pourcentage en poids supérieur à 90 % du poids total et de l'ail violet dans un pourcentage en poids compris entre 0,5 et 1,0 % du poids total, et dans laquelle les autres composants sont de la silice, du chlorure de sodium et des acidifiants jusqu'à ce que 100 % du poids total soit atteint, dans laquelle le noyau d'olive a une taille des particules comprise entre 5 et 7 mm.

2. Composition selon la revendication 1, dans laquelle le pourcentage en poids d'ail violet est compris entre 0,5 et 0,7 % p/p.

3. Composition selon la revendication précédente, dans laquelle le pourcentage en poids d'ail violet est de 0,65 % p/p.

4. Composition selon l'une quelconque des revendications précédentes, dans laquelle le pourcentage en poids de silice est compris entre 0,2 et 0,3 % p/p.

5. Composition selon la revendication précédente, dans laquelle le pourcentage en poids de silice est de 0,28 % p/p.

6. Composition selon l'une quelconque des revendications précédentes, dans laquelle le pourcentage en poids de chlorure de sodium est compris entre 0,02 et 0,07 % p/p.

7. Composition selon la revendication précédente, dans laquelle le pourcentage en poids de chlorure de sodium est de 0,05 % p/p.

8. Composition selon l'une quelconque des revendications précédentes, dans laquelle l'acidulant est choisi parmi l'acide citrique et l'extrait d'acide citrique.

9. Composition selon la revendication précédente, dans laquelle l'acidulant est une combinaison d'acide citrique et d'extrait d'acide citrique.

10. Composition selon la revendication 8, dans laquelle le pourcentage en poids d'acide citrique est compris entre 0,005 et 0,015 % p/p.

11. Composition selon la revendication précédente, dans laquelle le pourcentage en poids d'acide citrique est de 0,01 % p/p.

12. Composition selon la revendication 8, dans laquelle le pourcentage en poids d'extrait d'acide citrique est compris entre 0,005 et 0,015 % p/p.

13. Composition selon la revendication précédente, dans laquelle le pourcentage en poids d'extrait d'acide citrique est de 0,01 % p/p.

14. Utilisation d'une composition selon l'une quelconque des revendications 1 à 13 pour nettoyer les conduites d'alimentation d'exploitations agricoles.
